(19) Europäisches Patentamt
European Patent Office
Office européen des brevets

(11) Veröffentlichungsnummer: **0 220 614 B1**

# (12) EUROPÄISCHE PATENTSCHRIFT

(45) Veröffentlichungstag der Patentschrift: **23.01.91 Patentblatt 91/04**

(51) Int. Cl.[5]: **A61K 7/13**, C07C 229/42, C07C 309/40

(21) Anmeldenummer: **86114302.2**

(22) Anmeldetag: **16.10.86**

(54) **Haarfärbemittel mit direktziehenden Nitrodiphenylamin-Derivaten.**

(30) Priorität: **24.10.85 DE 3537765**

(43) Veröffentlichungstag der Anmeldung: **06.05.87 Patentblatt 87/19**

(45) Bekanntmachung des Hinweises auf die Patenterteilung: **23.01.91 Patentblatt 91/04**

(84) Benannte Vertragsstaaten: **AT BE CH DE FR GB IT LI NL SE**

(56) Entgegenhaltungen:
**DE-B- 2 830 497**
**FR-A- 2 216 271**
**CHEMICAL ABSTRACTS, Band 72, 1970, Seite 350, Zusammenfassung Nr. 110950d, Columbus, Ohio, US; P.T. FRANGOPOL et al.: "Stable diaryl-nitrogen free radicals with a sulfonic group in the molecule"**

(56) Entgegenhaltungen:
**CHEMICAL ABSTRACTS, Band 88, 1978, Seite 487, Zusammenfassung Nr. 89301m, Columbus, Ohio, US; I.S. SHUL'GA et al.: "Derivatives of 4,6-dinitrodiphenylamine-2-carboxylic acid, their synthesis and properties"**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Rose, David, Dr.**
**Holbeinweg 7**
**D-4010 Hilden (DE)**
Erfinder: **Lieske, Edgar**
**Hunsrückenstrasse 40**
**D-4000 Düsseldorf (DE)**
Erfinder: **Maak, Norbert, Dr.**
**Im Jagdfeld 41 a**
**D-4040 Neuss (DE)**

EP 0 220 614 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Gegenstand der Erfindung sind Haarfärbemittel mit einem Gehalt an direktziehenden Haarfarbstoffen. Solche Haarfärbemittel enthalten direktziehende Haarfarbstoffe in einem kosmetischen Träger. Oft enthalten solche Haarfärbemittel zur Erzielung bestimmter Nuancen zusätzlich Oxidationsfarbstoffvorprodukte. Als kosmetische Träger für die direktziehenden Haarfarbstoffe und gegebenenfalls Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Unter den direktziehenden Nitrobenzolderivaten spielen die Nitroaniline und deren Derivate eine besondere Rolle, da einige dieser Farbstoffe intensive Färbungen von guter Lichtechtheit ausbilden. Ein Nachteil der bekannten direktziehenden Nitroanilin-Farbstoffe ist jedoch einerseits eine begrenzte Wasserlöslichkeit, die zu Problemen bei der Formulierung der Haarfärbemittel führt, andererseits die unbefriedigende Waschechtheit, d. h. daß die Haarfärbungen nach mehrmaligem Haarwaschen stark ausbluten. Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Es wurde gefunden, daß Haarfärbemittel, die als direktziehende Haarfarbstoffe Verbindungen der allgemeinen Formel I

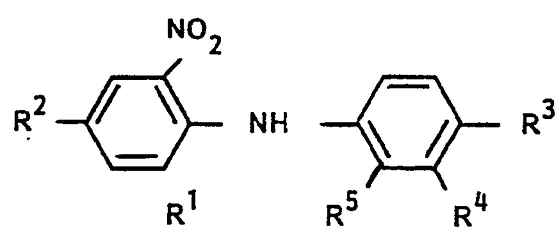

(I)

in der eine der Gruppen $R^1$ oder $R^2$ eine Nitrogruppe und die andere eine $-SO_3H$ oder -COOH-Gruppe ist, und die Gruppen $R^3$ bis $R^5$ unabhängig voneinander Wasserstoff, Alkylgruppen mit 1 bis 4 C-Atomen oder eine der Gruppen $R^3$ bis $R^5$ eine Hydroxylgruppe oder eine Aminogruppe der Formel $NR^6R^7$ ist, in der $R^6$ und $R^7$ Wasserstoff, Alkylgruppen mit 1 bis 4 C-Atomen oder Hydroxyalkylgruppen mit 2 bis 4 C-Atomen sind, und die beiden anderen Wasserstoff oder Alkylgruppen mit 1 bis 4 C-Atomen oder die eine Wasserstoff oder eine Alkylgruppe mit 1 bis 4 C-Atomen und die andere eine Hydroxymethylgruppe, eine Alkoxygruppe mit 1 bis 4 C-Atomen oder Chlor ist, oder deren wasserlösliche Salze enthalten sind, die gestellten Anforderungen in hohem Maße erfüllen.

Die Farbstoffe der allgemeinen Formel I erzeugen auf dem Haar gelbe bis kupferfarbige Nuancen von hoher Intensität, Licht- und Waschechtheit. Sie besitzen darüber hinaus im Vergleich zu bekannten Nitroanilinfarbstoffen eine bessere Löslichkeit im wäßrig-alkalischen Medium. In dermatologischer und toxikologischer Hinsicht sind die Verbindungen der Formel I unschädlich und daher für die Anwendung in Haarfärbemitteln besonders geeignet.

Verbindungen der allgemeinen Formel I, in der $R^3$, $R^4$ und $R^5$ Wasserstoff ist, sind literaturbekannt. Die Herstellung der übrigen Verbindungen der allgemeinen Formel I erfolgt allgemein durch Umsetzung eines 1-Chlor-2-nitrobenzolderivats der Formel II

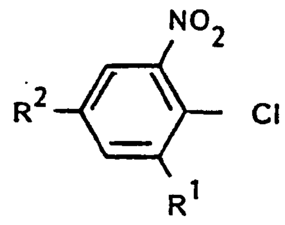

(II)

mit einem Anilinderivat der Formel III

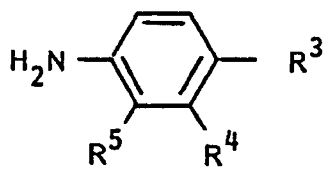

(III)

unter Abspaltung von HCl in Gegenwart einer Base.

Dabei haben die Gruppen $R^1$ bis $R^5$ die für die Formel I angegebene Bedeutung.

Verbindungen der Formel I, in der die Gruppe $R^3$ eine Hydroxylgruppe oder eine Aminogruppe $-NR^6R^7$, wobei $R^6$ und $R^7$ die vorgenannte Bedeutung haben, sind besonders geeignet und nicht literaturbekannt.

Ein weiterer Erfindungsgegenstand sind daher Verbindungen der Formel I, in der eine der Gruppen $R^1$ oder $R^2$ eine Nitrogruppe und die andere eine $-SO_3H$ oder -COOH-Gruppe ist und die Gruppe $R^3$ eine Hydroxylgruppe oder eine Aminogruppe-$NR^6R^7$ ist, in der $R^6$ und $R^7$ Wasserstoff, Alkylgruppen mit 1 bis 4 C-Atomen oder Hydroxyalkylgruppen mit 2 bis 4 C-Atomen sind und $R^4$ und $R^5$ Wasserstoff oder Alkylgruppen mit 1 bis 4 C-Atomen sind oder eine der Gruppen $R^4$ oder $R^5$ Wasserstoff oder eine Alkylgruppe mit 1 bis 4 C-Atomen und die andere eine Hydroxymethylgruppe, eine Alkoxygruppe mit 1 bis 4 C-Atomen oder Chlor ist und deren wasserlösliche Salze.

Unter wasserlöslichen Salzen sind in erster Linie die Salze starker Basen wie etwa die Alkalisalze, z. B. das Natrium- oder Kaliumsalz oder Ammoniumsalze, Alkanolammoniumsalze mit 2 bis 4 C-Atomen in der Alkanolgruppe wie z. B. das Monoethanolammoniumsalz, das Triethanolammoniumsalz oder das Isopropanolammoniumsalz zu verstehen. Die erfindungsgemäßen Haarfärbemittel können die direktziehenden Nitrodiphenylaminderivate der allgemeinen Formel I allein oder in Kombination mit bekannten direktziehenden Farbstoffen, z. B. mit anderen Nitrobenzolderivaten, Anthrachinonfarbstoffen, Triphenylmethan- oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl-oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt ; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 bis 2 Gew.-%, bezogen auf das gesamte Haarfärbemittel, eingesetzt. Daneben können bekannte Oxidationshaarfärbemittelvorprodukte (Entwickler- und Kupplersubstanzen) in einer Menge von 0,01 bis 5, vorzugsweise von 1 bis 3 Gew.-% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

## Beispiele

### Herstellungsbeispiele

1. 2,6-Dinitro-4'-dimethylamino-diphenylamin-4-sulfonsäure-K-Salz

Eine Mischung aus 5 g 4-Chlor-3,5-dinitrobenzolsulfonsäure-K-Salz (0,016 Mol), 2,2 g N,N-Dimethyl-p-phenylendiamin (0,016 Mol) und 1,1 g Kaliumcarbonat (0,008 Mol) in 50 ml Wasser wurde 1 Stunde unter Rückfluß gekocht. Nach dem Abkühlen wurde der Niederschlag abfiltriert. Es wurde ein ockerbraunes Pulver erhalten.
UV-Spektrum (pH = 9,5) $\lambda_{MAX}$ = 437 nm

2. 2,6-Dinitro-4'-amino-diphenylamin-4-sulfonsäure-K-Salz

Die Herstellung erfolgte analog Beispiel 1. Anstatt N,N-Dimethyl-p-phenylendiamin wurden 1,73 g p-Phenylendiamin (0,016 Mol) eingesetzt.
UV-Spektrum (pH = 9,5) $\lambda_{MAX}$ = 431 nm

3. 2,6-Dinitro-4'-methylamino-diphenylamin-4-sulfonsäure-K-Salz

Die Herstellung erfolgte analog Beispiel 1. Anstatt N,N-Dimethyl-p-phenylendiamin wurden 3,1 g N-Methyl-p-phenylendiamin (0,016 Mol) eingesetzt.
UV-Spektrum (pH = 9,5) $\lambda_{MAX}$ = 441 nm

4. 2,6-Dinitro-2'-methyl-4'-amino-diphenylamin-4-sulfonsäure-K-Salz

Die Herstellung erfolgte analog Beispiel 1. Anstatt N,N-Dimethyl-p-phenylendiamin wurden 3,5 g p-Toluylendiaminsulfat (0,016 Mol) eingesetzt. Rotbraunes Pulver.
UV-Spektrum (pH = 9,5) $\lambda_{MAX}$ = 449 nm

5. 2,6-Dinitro-4'-N,N-bis-(β-hydroxyethyl)-amino-diphenylamin-4-sulfonsäure-K-Salz

Die Herstellung erfolgte analog Beispiel 1. Anstatt N,N-Dimethyl-p-phenylendiamin wurden 5,2 g N,N-Bis-(β-hydroxyethyl)-p-phenylendiamin (0,016 Mol) eingesetzt. Rotbraunes Pulver.
UV-Spektrum (pH = 9,5) $\lambda_{MAX}$ = 443 nm

6. 2,6-Dinitro-2′-chlor-4′-amino-diphenylamin-4-sulfonsäure-K-Salz

Die Herstellung erfolgte analog Beispiel 1. Anstatt N,N-Dimethyl-p-phenylendiamin wurden 3,85 g 2-Chlor-p-phenylen-diaminsulfat (0,016 Mol) eingesetzt. Rotbraune Kristalle.
IR-Spektrum (KBr ; $cm^{-1}$) ; 1620, 1565, 1530, 1505, 1420, 1340, 1270, 1240, 1205, 1150, 1130, 1110, 1045, 920, 910, 895, 825

7. 2,6-Dinitro-2′,3′-dimethyl-4′-amino-diphenylamin-4-sulfonsäure-K-Salz

Die Herstellung erfolgte analog Beispiel 1. Anstatt N,N-Dimethyl-p-phenylendiamin wurden 2,2 g (0,016 Mol) 2,3-Dimethyl-p-phenylendiamin eingesetzt. Braune Kristalle.
IR-Spektrum (KBr ; $cm^{-1}$) ; 1620, 1565, 1530, 1490, 1420, 1345, 1270, 1210, 1180, 1105, 1050, 930, 905, 835, 820

8. 2,6-Dinitro-3′-hydroxymethyl-4′-amino-diphenylamin-4-sulfonsäure-K-Salz

Die Herstellung erfolgte analog Beispiel 1. Anstatt N,N-Dimethyl-p-phenylendiamin wurden 3,8 g 2,5-Diaminobenzylalkohol (0,016 Mol) eingesetzt. Braune Kristalle.
UV-Spektrum (pH = 9,5) $\lambda_{MAX}$ = 427 nm

9. 2,6-Dinitro-2′-methoxy-4′amino-diphenylamin-4-sulfonsäure-K-Salz

Die Herstellung erfolgte analog Beispiel 1. Anstatt N,N-Dimethyl-p-phenylendiamin wurden 3,8 g 2,5-Diaminoanisolsulfat (0,016 Mol) eingesetzt. Braune Kristalle.
UV-Spektrum (pH = 9,5) $\lambda_{MAX}$ = 424 nm

10. 2,6-Dinitro-4′-hydroxy-diphenylamin-4-sulfonsäure-K-Salz

Die Herstellung erfolgte analog Beispiel 1. Anstatt N,N-Dimethyl-p-phenylendiamin wurden 1,75 g p-Aminophenol (0,016 Mol) eingesetzt.

11. 2,6-Dinitro-4′-N-ethyl-N-β-hydroxyethylamino-diphenylamin-4-sulfonsäure-K-Salz

Die Herstellung erfolgte analog Beispiel 1. Anstatt N,N-Dimethyl-p-phenylendiamin wurden 4,5 g N-Ethyl-N-β-hydroxyethyl-p-phenylendiaminsulfat eingesetzt. Schwarze Kristalle.
UV-Spektrum (pH = 9,5) $\lambda_{MAX}$ = 446 nm

12. 2,6-Dinitro-2′-methyl-4′-N,N-diethylamino-diphenylamin-4-sulfonsäure-K-Salz

Die Herstellung erfolgte analog Beispiel 1. Anstatt N,N-Dimethyl-p-phenylendiamin wurden 3,4 g N,N-Diethyl-2-methyl-p-phenylendiamin-dihydrochlorid (0,016 Mol) eingesetzt. Schwarze Kristalle.
IR-Spektrum (KBr ; $cm^{-1}$) ; 1620, 1565, 1510, 1470, 1450, 1430, 1400, 1380, 1355, 1340, 1265, 1240, 1200, 1115, 1100, 1080, 1045, 930, 905, 870, 840.

13. 2,4-Dinitro-4′-dimethylamino-diphenylamin-4-carbonsäure

Eine Mischung bestehend aus 5 g N,N-Dimethyl-p-phenylendiamin (0,024 Mol), 6 g 3,5-Dinitro-2-chlorbenzoesäure (0,024 Mol) und 3,3 g Kaliumcarbonat in 40 ml Wasser wurde 2 Stunden bei Zimmertemperatur gerührt. Der Niederschlag wurde abfiltriert. Curryfarbene Kristalle.
Schmelzpunkt 249°C (unter Zersetzung)

14. 2,6-Dinitro-4′-amino-diphenylamin-4-carbonsäure

Eine Mischung bestehend aus 5 g 4-Chlor-3,5-dinitrobenzoesäure (0,024 Mol), 3 g p-Phenylendiamin (0,024 Mol) und 5,4 g Natriumacetat, wurde in 60 ml Wasser unter Rückfluß gekocht. Nach dem Abkühlen

wurde der Niederschlag abfiltriert und mehrmals mit Wasser ausgekocht. Rückstand : braune Kristalle. Schmelzpunkt 160°C (unter Zersetzung)

15. 2,6-Dinitro-4′-amino-3′-methyl-diphenylamin-4-carbonsäure

Die Herstellung erfolgte analog Beispiel 14. Anstatt p-Phenylendiamin wurden 4,4 g p-Toluylendiaminsulfat (0,02 Mol) eingesetzt. Hellbraune Kristalle.
Schmelzpunkt 188°C (unter Zersetzung)

16. 2,6-Dinitro-4′-N,N-dimethylamino-diphenylamin-4-carbonsäure

Die Herstellung erfolgte analog Beispiel 14. Anstatt p-Phenylendiamin wurden 5 g N,N-Dimethyl-p-phenylendiamindihydrochlorid (0,02 Mol) eingesetzt. Schwarze Kristalle.
Schmelzpunkt 228 bis 232°C

17. 2,6-Dinitro-4′-hydroxy-diphenylamin-4-carbonsäure

Die Herstellung erfolgte analog Beispiel 14. Anstatt p-Phenylendiamin wurden 2,18 g p-Aminophenol (0,02 Mol) eingesetzt. Orangebraune Kristalle.
Schmelzpunkt 240°C

18. 2,4-Dinitro-4′-amino-diphenylamin-6-sulfonsäure-Na-Salz

Eine Mischung, bestehend aus 10 g 2-chlor-3,5-dinitrobenzolsulfonsäure-Na-Salz (0,034 Mol), 3,7 g p-Phenylendiamin (0,034 Mol) und 2,4 g Kaliumcarbonat (0,017 Mol) in 75 ml Wasser wurde 2 Stunden unter Rückfluß gekocht. Nach dem Abkühlen wurde der Niederschlag abfiltriert. Rotbraune Kristalle.
Schmelzpunkt oberhalb 305°C

19. 2,4-Dinitro-2′-chlor-4′-amino-diphenylamin-6-sulfonsäure-Na-Salz

Die Herstellung erfolgte analog Beispiel 18. Anstatt p-Phenylendiamin wurden 4,1 g 2-Chlor-p-phenylendiamin (0,017 Mol) eingesetzt. Braune Kristalle.
Schmelzpunkt oberhalb 305°C

20. 2,4-Dinitro-4′-hydroxy-diphenylamin-6-sulfonsäure-Na-Salz

Die Herstellung erfolgte analog Beispiel 18. Anstatt p-Phenylendiamin wurden 1,86 g p-Aminophenol (0,017 Mol) eingesetzt. Braune Kristalle.
Schmelzpunkt oberhalb 305°C

21. 2,4-Dinitro-4′-N-ethyl-N-β-hydroxyethyl-aminodiphenylamin-6-sulfonsäure-Na-Salz

Die Herstellung erfolgte analog Beispiel 18. Anstatt p-Phenylendiamin wurden 4,7 g N-Ethyl-N-β-hydroxy-ethyl-p-phenylendiamin (0,017 Mol) eingesetzt. Schwarze Kristalle.
Schmelzpunkt oberhalb 160°C (unter Zersetzung)

22. 2,4-Dinitro-2′-methyl-4′-amino-diphenylamin-6-sulfonsäure-Na-Salz

Die Herstellung erfolgte analog Beispiel 18. Anstatt p-Phenylendiamin wurden 3,75 g p-Toluylendiaminsulfat (0,017 Mol) eingesetzt. Rotbraune Kristalle.
Schmelzpunkt oberhalb 305°C

23. 2,4-Dinitro-4′-N,N-bis-(β-hydroxyethyl)-amino-diphenylamin-6-sulfonsäure-Na-Salz

Die Herstellung erfolgte analog Beispiel 18. Anstatt p-Phenylendiamin wurden 4,6 g N,N-bis-(β-hydroxyethyl)-p-phenylendiamin (0,017 Mol) eingesetzt. Schwarze Kristalle.
Schmelzpunkt 174°C

Folgende literaturbekannte Verbindungen wurden zusätzlich in die Haarfärbeversuche einbezogen :

24. 2,4-Dinitrodiphenylamin-6-sulfonsäure-Na-Salz

(Ann. 366, 83 (1909))

25. 2,6-Dinitro-diphenylamin-4-carbonsäure

(Am. Chem. J. 19, 18 (1897))

26. 2,6-Dinitro-diphenylamin-4-sulfonsäure

(Ann. 366, 106 (1909))

Haarfärbeversuche

Es wurden Haarfärbecremes der folgenden Zusammensetzung hergestellt :

| | |
|---|---|
| Fettalkohol $C_{12-18}$ | 10 g |
| Fettalkohol $C_{12-14}$ + 2 EO-sulfat, Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90% ergrauten, aber nicht besonders vorbehandelten Menschenhaars aufgetragen und dort 30 Minuten bei 27°C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiel 1 bis 26 eingesetzt.

Das Ergebnis der Färbeversuche ist der Tabelle I zu entnehmen.

Tabelle I

| direktziehender Farbstoff gemäß Beispiel | Nuance |
|---|---|
| 1 | braun |
| 2 | orangebraun |
| 3 | braun |
| 4 | orangebraun |
| 5 | braun |
| 6 | orangebraun |
| 7 | orangebraun |
| 8 | kastanienbraun |
| 9 | hellbraun |
| 10 | apfelsinengelb |
| 11 | braun |
| 12 | haarbraun |
| 13 | gelb |
| 14 | braun |
| 15 | braun |
| 16 | braun |
| 17 | gelb |
| 18 | topasgelb |
| 19 | aprikosengelb |
| 20 | goldgelb |
| 21 | hellbraun |
| 22 | kupferfarbig |
| 23 | cognacbraun |
| 24 | olivgelb |
| 25 | tiefgelb |
| 26 | gelb |

## Ansprüche

1. Verwendung von Verbindungen der Formel I

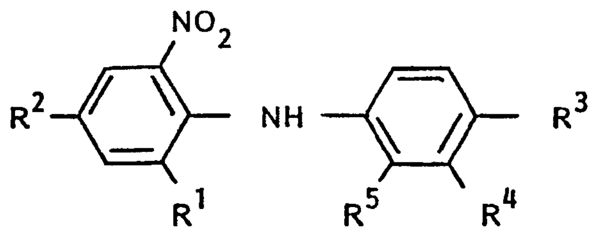

(I)

in der eine der Gruppen $R^1$ oder $R^2$ eine Nitrogruppe und die andere eine $-SO_3H$ oder -COOH-Gruppe ist, und die Gruppen $R^3$ bis $R^5$ unabhängig voneinander Wasserstoff, Alkylgruppen mit 1 bis 4 C-Atomen oder eine der Gruppen $R^3$ bis $R^5$ eine Hydroxygruppe oder eine Aminogruppe der Formel $NR^6R^7$ ist, in der $R^6$ und $R^7$ Wasserstoff, Alkylgruppen mit 1 bis 4 C-Atomen oder Hydroxyalkylgruppen mit 2 bis 4 C-Atomen sind, und die beiden anderen Wasserstoff oder Alkylgruppen mit 1 bis 4 C-Atomen oder die eine Wasserstoff oder eine Alkylgruppe mit 1 bis 4 C-Atomen und die andere eine Hydroxymethylgruppe, eine Alkoxygruppe mit 1 bis 4 C-Atomen oder Chlor ist, oder deren wasserlösliche Salze als direktziehende Haarfarbstoffe zur Herstellung von Haarfärbemitteln.

2. Verwendung nach Patentanspruch 1, dadurch gekennzeichnet, daß die Gruppe $R^3$ eine Hydroxylgruppe oder eine Aminogruppe $-NR^6R^7$ ist, wobei $R^6$ und $R^7$ die in Anspruch 1 angegebene Bedeutung haben.

3. Haarfärbemittel mit einem Gehalt an direktziehenden Haarfarbstoffen in einem kosmetischen Träger auf Basis üblicher Bestandteile solcher kosmetischer Zubereitungen, dadurch gekennzeichnet, daß als direktziehende Haarfarbstoffe Verbindungen der Formel I gemäß Patentanspruch 1 oder 2 in einer Menge von 0,01 bis 5 Gew.-%, bezogen auf das gesamte Haarfärbemittel, enthalten sind.

## Claims

1. The use of compounds corresponding to formula I

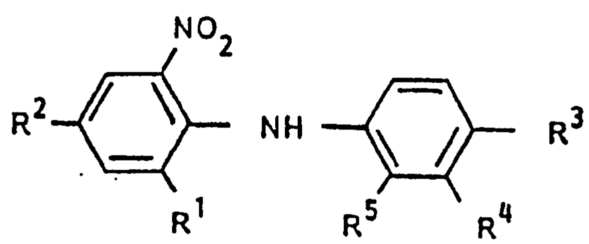

(I)

in which one of the groups $R^1$ or $R^2$ is a nitro group and the other is an $-SO_3H$ or -COOH- group and the groups $R^3$ to $R^5$ independently of one another represent hydrogen, $C_1$-$C_4$ alkyl groups or one of the groups $R^3$ to $R^5$ is a hydroxy group or an amino group of the formula $NR^6R^7$, where $R^6$ and $R^7$ represent hydrogen, $C_1$-$C_4$ alkyl groups or $C_2$-$C_4$ hydroxyalkyl groups, and the other two represent hydrogen or $C_1$-$C_4$ alkyl groups or one represents hydrogen or a $C_1$-$C_4$ alkyl group and the other a hydroxymethyl group, a $C_1$-$C_4$ alkoxy group or chlorine,

or water-soluble salts thereof as substantive hair dyes for the production of hair dyeing preparations.

2. The use claimed in claim 1, characterized in that the group $R^3$ is a hydroxyl group or an amino group $-NR^6R^7$, where $R^6$ and $R^7$ are as defined in Claim 1.

3. Hair-dyeing preparations containing substantive hair dyes in a cosmetic carrier based on typical constituents of such cosmetic preparations, characterized in that compounds of formula I according to claim 1 or 2 are present as the substantive dyes in a quantity of from 0.01 to 5% by weight, based on the hair-dyeing preparation as a whole.

## Revendications

1. Mise en oeuvre de composés de la formule I

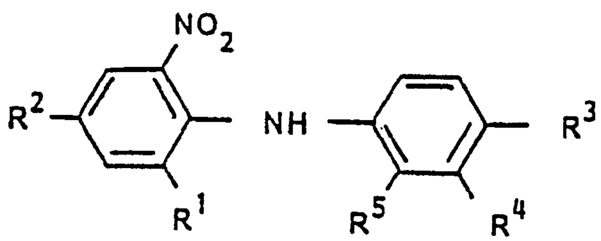

(I)

dans laquelle un des groupes $R^1$ ou $R^2$ est un groupe nitro et l'autre un groupe $-SO_3H$ ou -COOH, et dans laquelle les groupes $R^3$ à $R^5$, indépendamment l'un de l'autre, sont de l'hydrogène, des groupes alkyle avec 1 à 4 atomes C ou bien dans laquelle un des groupes $R^3$ à $R^5$ est un groupe hydroxy ou un groupe amino de la formule $NR^6R^7$, où $R^6$ et $R^7$ sont de l'hydrogène, des groupes alkyle avec 1 à 4 atomes C ou des groupes hydroxyalkyle avec 2 à 4 atomes C, et les deux autres de l'hydrogène ou des groupes alkyle avec 1 à 4 atomes C, ou l'un de l'hydrogène ou un groupe alkyle avec 1 à 4 atomes C et l'autre un groupe hydroxyméthyle, un groupe alkoxy avec 1 à 4 atomes C ou du chlore, ou leurs sels solubles dans l'eau, en guise de colorants capillaires montant directement sur la fibre pour la fabrication de teintures capillaires.

2. Utilisation selon la formule I, caractérisée en ce que le groupe $R^3$ est un groupe hydroxyle ou un groupe amino $-NR^6R^7$, où $R^6$ et $R^7$ ont la signification indiquée à la revendication 1.

3. Teintures capillaires contenant des colorants montant directement sur la fibre dans un support cosmétique à base des éléments courants dans de telles préparations cosmétiques, caractérisées en ce qu'elles contiennent, comme colorants capillaires montant directement sur la fibre, des composés de la formule I selon la revendication 1 ou 2, dans une proportion de 0,01 à 5% en poids par rapport à l'ensemble de la teinture capillaire.